# EUROPEAN PATENT SPECIFICATION

(11) **EP 4 169 494 B1**
(45) Date of publication and mention of the grant of the patent: **10.04.2024**
(21) Application number: 22201609.9
(22) Date of filing: 14.10.2022
(51) Int. Cl.: A61F 13/42, A61F 13/495, G01N 27/04

(54) **STOOL DETECTION DEVICE**
STUHLERKENNUNGSVORRICHTUNG
DISPOSITIF DE DÉTECTION DE SELLES

(30) Priority: 19.10.2021 TW 110212281 U
(43) Date of publication of application: 26.04.2023
(73) Proprietor: I-DING MEDICAL EQUIPMENT CO. LTD., Kaohsiung City 81352 (TW)
(72) Inventor: CHEN, Hung-Chi, 81352 Kaohsiung City (TW)
(74) Representative: Zimmermann, Tankred Klaus

(56) References cited:
- US-A1- 2012 245 542

## Description

The disclosure relates to a detection device, and more particularly to a stool detection device.

Many elderly patients in care homes wear diapers and are cared for by caregivers. Although technological progress have led to the development of wetness indicators on the diapers so that a caregiver may be easily notified of a patient's need to change diapers, the applications of the wetness indicator are still limited, since the bodily functions of the patient include the need to both urinate and defecate. In cases where the patient defecates, the wetness indicator on the diaper worn by the patient may not reliably indicate the need to change diapers. Additionally, failure to promptly change the soiled diaper may cause significant health issues for the patient and affect the patient's quality of life.

Therefore, an object of the disclosure is to provide a stool detection device that can alleviate at least one of the drawbacks of the prior art.

According to the disclosure, a stool detection device is adapted for detecting and notifying defecation of stool in a diaper. The diaper has an inner surface and an outer surface, and is formed with a through hole through the inner surface and the outer surface. The stool detection device is adapted to communicate with a receiver, and includes a positioning member, a mounting seat, a transmitter module and a stool detector.

The positioning member is adapted to be removably mounted to the diaper, and to extend through the through hole of the diaper.

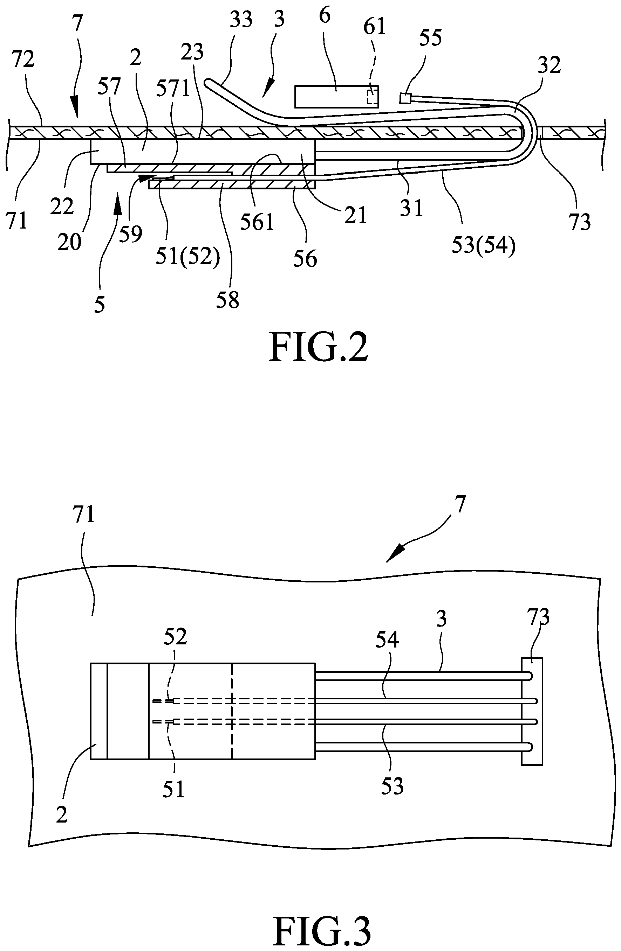

The mounting seat is mounted to the positioning member in a manner that the mounting seat is disposed inside the diaper when the positioning member is mounted to the diaper. The mounting seat has a mounting surface, an abutting member, a first end and a second end. The mounting surface is adapted to face away from the inner surface of the diaper when the positioning member is mounted to the diaper. The abutting surface is adapted to abut against the inner surface of the diaper when the positioning member is mounted to the diaper. The first end is connected to the positioning member, and the second end is opposite to the first end in a first direction (i.e., a left-right direction in Figure 2) parallel to the mounting surface.

The transmitter module is removably mounted on the positioning member in a manner that the transmitting module is disposed outside the diaper when the positioning member is mounted on the diaper.

The stool detector is mounted on the mounting surface of the mounting seat, and includes a main body, a long board-member, a short board-member, a first conductive sheet, a second conductive sheet, a first electric wire and a second electric wire. The main body is made of electrically insulating material, is adjacent to the first end of the mounting seat, has a first adhesive surface removably adhered to the mounting surface of the mounting seat, and is coated with hydrophobic material except for on the first adhesive surface. The long board-member is made of electrically insulating material, extends from the main body towards the second end of the mounting seat, has a second adhesive surface removably adhered to the mounting seat surface of the mounting seat, and is coated with hydrophobic material except for on the second adhesive surface. The short board-member is made of an electrically insulating material, extends from the main body towards the second end of the mounting seat, is spaced apart from the long board-member, is thinner than the main body in a second direction perpendicular to the first direction, co-operates with the main body and the long board-member to define a receiving space, and is coated with hydrophobic material. The first conductive sheet is disposed in the receiving space. The second conductive sheet is disposed in the receiving space, and is spaced apart from the first conductive sheet. The first electric wire is adapted to extend through the through hole of the diaper, and has one end electrically connected to the first conductive sheet, and the other end electrically connected to the transmitter module in a removable connection. The second electric wire is adapted to extend through the through hole of the diaper, and has one end electrically connected to the second conductive sheet and the other end electrically connected to the transmitter module in a removable connection.

When the stool gets into the receiving space of the stool detector and contacts both the first conductive sheet and the second conductive sheet to establish an electrical connection between the first conductive sheet and the second conductive sheet, the transmitter module is configured to be activated to wirelessly transmit a detection signal to the receiver to allow the receiver to output a notification.

Other features and advantages of the disclosure will become apparent in the following detailed description of the embodiment with reference to the accompanying drawings, of which:
Figure 1 is a schematic view illustrating an embodiment of a stool detection device according to the disclosure;
Figure 2 is a fragmentary sectional view illustrating the embodiment mounted to a diaper;
Figure 3 is a schematic view of the embodiment of the stool detection device; and
Figure 4 is a schematic top view showing the embodiment where stool gets in a receiving space of a stool detector of the stool detection device.

Before the disclosure is described in greater detail, it should be noted that where considered appropriate, reference numerals or terminal portions of reference numerals have been repeated among the figures to indicate corresponding or analogous elements, which may optionally have similar characteristics.

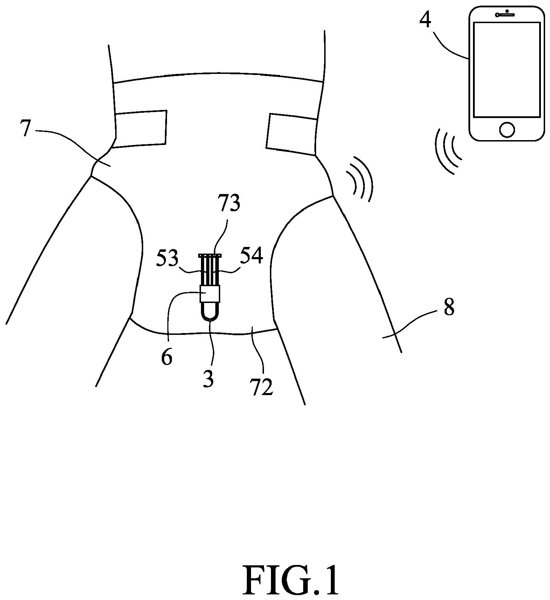

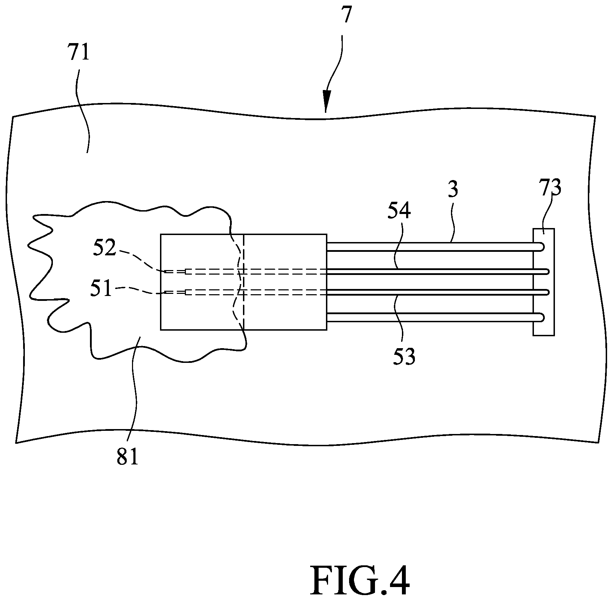

Referring to Figures 1 to 4, an embodiment of a stool detection device according to this disclosure is shown. The stool detection device is adapted for detecting and notifying defecation of stool 81 in a diaper 7 worn by a wearer 8. The diaper 7 has an inner surface 71 and an outer surface 72, and is formed with a through hole 73 through the inner surface 71 and the outer surface 73. In this embodiment the stool detection device includes a mounting seat 2, a positioning member 3, a transmitter module 6 and a stool detector 5. The stool detection device is adapted to communicate with a receiver 4. In this embodiment the receiver 4 may be a portable device such as a smartphone or a tablet.

Referring to Figure 2, the positioning member 3 is adapted to be removably mounted to the diaper 7 and to extend through the through hole 73 of the diaper 7. The mounting seat 2 is mounted to the positioning member 3 in a manner that the mounting seat 2 is disposed inside the diaper 7 when the positioning member 3 is mounted to the diaper 7. The mounting seat 2 has a mounting surface 20 adapted to face away from the inner surface 71 of the diaper 7 when the positioning member 3 is mounted to the diaper 7, an abutting surface 23 adapted to abut against the inner surface 71 of the diaper 7 when the positioning member 3 is mounted to the diaper 7, a first end 21 connected to the positioning member 3, and a second end 22 opposite to the first end 21 in a first direction parallel to the mounting surface 20.

In this embodiment, the positioning member 3 may be a clip as shown in Figure 2, and includes a first potion 31 that is partially inserted into the first end 21 of the mounting seat 2, a second portion 32 that curvedly extends from the first portion 31, and a third portion 33 that extends from the second portion 32. Specifically, when the positioning member 3 clips to the diaper 7, the first portion 31 is disposed on the inner surface 71 of the diaper 7, the mounting surface 20 of the mounting seat 2 faces away from the inner surface 71 of the diaper 7, the second portion 32 extends across the through hole 73, and the third portion 33 abuts against the outer surface 72 of the diaper 7.

The transmitter module 6 is removably mounted to the positioning member 3 in a manner that the transmitter module 6 is disposed outside the diaper 7 when the positioning member 3 is mounted on the diaper 7, and is configured to wirelessly communicate with the receiver 4. More specifically, the transmitter module 6 is removably mounted to the third portion 33 of the positioning member 3, and includes an input port 61 and other components such as a processor, a wireless transmitter, a battery, etc. (not shown). For example, the transmitter module 6 may be attached to the third portion 33 of the positioning member 3 by magnets, hook-and-loop fasteners, etc.

The stool detector 5 is mounted on the mounting surface 20 of the mounting seat 2, and includes a main body 56, a long board-member 57, a short board-member 58, a first conductive sheet 51, a second conductive sheet 52, a first electric wire 53, a second electric wire 54 and a connector 55.

In this embodiment the main body 56 of the stool detector 5 is made of electrically insulating material, is adjacent to the first end 21 of the mounting seat 2, and has a first adhesive surface 561 removably adhered to the mounting surface 20 of the mounting seat 2. More specifically, the first adhesive surface 561 is adhered to the mounting surface 20 adjacent to the first end 21 of the mounting seat. The long board-member 57 is made of electrically insulating material, extends from the main body 56 towards the second end 22 of the mounting seat 2, and has a second adhesive surface 571 removably adhered to the mounting surface 20 of the mounting seat 2. The long board-member 57 is thinner than the main body 56 in a second direction (i.e., an up-down direction in Figure 2) perpendicular to the first direction. The short board-member 58 is made of electrically insulating material, extends from the main body 56 towards the second end 22 of the mounting seat 2, is spaced apart from the long board-member 57, is thinner than the main body 56 in the second direction, and co-operates with the main body 56 and the long board-member 57 to define a receiving space 59. More specifically, the thickness of the main body 56 is thicker than the thickness of the short board-member 58 in a second direction perpendicular to the first direction, and a length of the long board-member 57 is longer than a length of the short board-member 58 in the first direction. It should be noted that in this embodiment the main body 56, the long board-member 57 and the short board-member 58 of the stool detector 5 are formed as one piece. However, in other embodiments, the short board-member 58 may be formed with the main body 56 as one piece and the long board-member 57 is a separate component connected to the main body 56, or alternatively, the long board-member 57 may be formed with the main body 56 as one piece and the short board-member 58 is a separate component connected to the main body 56.

In this embodiment, the main body 56, the long board-member 57 and the short board-member 58 are all coated with hydrophobic material. More specifically, the main body 56 is coated with hydrophobic material except for on the first adhesive surface 561, and the long board-member 57 is coated with hydrophobic material except for on the second adhesive surface 571. When the wearer 8 wearing the diaper 7 mounted with the stool detection device urinates, the urine will be repelled by the hydrophobic material and form droplets, and thus the urine's contact with the short board-member 58, the long board-member 57 and the main body 56 will be minimised due to the coated hydrophobic material. That is to say the urine will not rest on the main body 56, the long board-member 57 and the short board-member 58.

The first conductive sheet 51 and the second conductive sheet 52 are both disposed in the receiving space 59, and are spaced apart from each other. The first electric wire 53 is adapted to extend through the through hole 73 of the diaper 7, and has one end electrically connected to the first conductive sheet 51 and the other end electrically connected to the transmitter module 6 in a removable connection. The second electric wire 54 is adapted to extend through the through hole 73 of the diaper 7, and has one end electrically connected to the second conductive sheet 52 and the other end electrically connected to the transmitter module 6 in a removable connection.

In this embodiment, the connector 55 is connected to said the other end of the first electric wire 53 and said the other end of the second electric wire 54 in a manner that said the other end of the first electric wire 54 and said the other end of the second electric wire 54 are insulated from each other. The connector 55 is removably inserted into the input port 61 of the transmitting module 6 so as to electrically connect both the first electric wire 53 and the second electric wire 54 to the transmitting module 6.

The transmitter 6 module is configured to use a wireless technology to transmit the detection signal to the receiver 4. Various wireless technologies may be used and the transmitter module 6 may use wireless technologies such as Wi-Fi, Bluetooth^{®} or radio-frequency identification, but is not limited to such.

In order to operate the stool detection device, a caregiver will adhere the stool detector 5 to the mounting surface 20 by adhering the first adhesive surface 561 of the stool detector 5 to the first end 21 of the mounting seat 2, and adhere the second adhesive surface 571 of the stool detector 5 to the second end 22 of the mounting seat 2. Next the caregiver will operate the third portion 33 of the positioning member 3, beginning from the inner surface 71 of the diaper 7 and threading the third portion 33 through the through hole 73 until the second portion 32 of the positioning member 3 extends across the through hole 73, and the third portion 33 abuts against the outer surface of the diaper 7. Through this operation, the other ends of both the first and second electric wires 53, 54 that extend along the positioning member 3 are also threaded through the through hole 73 to be on the outer surface 72 of the diaper 7. Afterwards the connector 55 is inserted into the input port 61 of the transmitter module 6, and the transmitter module 6 is removably disposed on the positioning member 3.

When the wearer 8 urinates, the urine will be repelled by the hydrophobic material coated on the short board-member 58, the long board-member 57 and the main body 56. Therefore, the urine will most likely be unable to form an electric connection between the first conductive sheet 51 and the second conductive sheet 52. Therefore, the stool detection device is specifically designed to detect the stool 81 of the wearer 8.

Referring to Figures 1, 2 and 4, when the wearer 8 wearing the diaper 7 mounted with a stool detection device defecates stool 81, the stool 81 gets in the receiving space 59 of the stool detector 5 and contacts both the first conductive sheet 51 and the second conductive sheet 52 to establish an electrical connection between the first conductive sheet 51 and the second conductive sheet 52. Accordingly, a closed circuit among components of the transmitter module 6 (e.g., the wireless transmitter, the battery, etc.) is formed due to the electrical connection between the first conductive sheet 51 and the second conductive sheet 52, and thus the transmitter module 6 is activated to wirelessly transmit a detection signal to the receiver 4 to allow the receiver 4 to output a notification. It should be noted that the stool 81 that gets in the receiving space 59 and establishes an electrical connection between the first conductive sheet 51 and the second conductive sheet 52 is optimally a type of stool that is moist and not too dry, specifically, stool type 4 to type 7 defined by the Bristol stool chart.

In this embodiment, the wearer 8 wearing the diaper 7 mounted with the stool detection device is cared for by a caregiver holding the receiver 4. Furthermore, in this embodiment, the receiver 4 is a smartphone with a defecation notification application. After the wearer 8 has defecated and the receiver 4 receives the detection signal sent from the transmitter module 6, the defecation notification application will notify the caregiver of the wearer's defecation so that the diaper 7 may be changed.

In summary of the above, the advantages and achievable effects of the stool detection device are that it can detect defecation of stool 81 by a wearer 8 wearing the diaper 7 mounted with the stool detection device, and the caregiver may be notified of the stool detection so that the diaper 7 may be changed.

In the description above, for the purposes of explanation, numerous specific details have been set forth in order to provide a thorough understanding of the embodiment. It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects, and that one or more features or specific details from one embodiment may be practiced together with one or more features or specific details from another embodiment, where appropriate, in the practice of the disclosure.

## Claims

1. A stool detection device adapted for detecting and notifying defecation of stool (81) in a diaper (7) that has an inner surface (71) and an outer surface (72), that is formed with a through hole (73) through the inner surface (71) and the outer surface (72), said stool detection device being adapted to communicate with a receiver (4) and **characterised by**:
a positioning member (3) being adapted to be removably mounted to the diaper (7) and to extend through the through hole (73) of the diaper (7);
a mounting seat (2) mounted to said positioning member (3) in a manner that said mounting seat (2) is disposed inside the diaper (7) when said positioning member (3) is mounted to the diaper (7), and having a mounting surface (20) adapted to face away from the inner surface (71) of the diaper (7) when said positioning member (3) is mounted to the diaper (7), an abutting surface (23) adapted to abut against the inner surface (71) of the diaper (7) when said positioning member (3) is mounted to the diaper (7), a first end (21) connected to said positioning member (3), and a second end (22) opposite to said first end (21) in a first direction parallel to said mounting surface (20);
a transmitter module (6) removably mounted on said positioning member (3) in a manner that said transmitting module (6) is disposed outside the diaper (7) when said positioning member (3) is mounted on the diaper (7); and
a stool detector (5) mounted on said mounting surface (20) of said mounting seat (2), and including
a main body (56) that is made of electrically insulating material, that is adjacent to said first end (21) of said mounting seat (2), that has a first adhesive surface (561) removably adhered to said mounting surface (20) of said mounting seat (2), and that is coated with hydrophobic material except for on said first adhesive surface (561),
a long board-member (57) that is made of electrically insulating material, that extends from said main body (56) towards said second end (22) of said mounting seat (2), that has a second adhesive surface (571) removably adhered to said mounting surface (20) of said mounting seat (2), and that is coated with hydrophobic material except for on said second adhesive surface (571),
a short board-member (58) that is made of an electrically insulating material, that extends from said main body (56) towards said second end (22) of said mounting seat (2), that is spaced apart from said long board-member (57), that is thinner than said main body (56) in a second direction perpendicular to the first direction, that co-operates with said main body (56) and said long board-member (57) to define a receiving space (59), and that is coated with hydrophobic material,
a first conductive sheet (51) that is disposed in said receiving space (59),
a second conductive sheet (52) that is disposed in said receiving space (59), and that is spaced apart from said first conductive sheet (51),
a first electric wire (53) that is adapted to extend through the through hole (73) of the diaper (7), and that has one end electrically connected to said first conductive sheet (51) and the other end electrically connected to said transmitter module (6) in a removable connection, and
a second electric wire (54) that is adapted to extend through the through hole (73) of the diaper (7), and that has one end electrically connected to said second conductive sheet (52) and the other end electrically connected to said transmitter module (6) in a removable connection,
wherein, when the stool (81) gets into said receiving space (59) of said stool detector (5) and contacts both said first conductive sheet (51) and said second conductive sheet (52) to establish an electrical connection between said first conductive sheet (51) and said second conductive sheet (52), said transmitter module (6) is configured to be activated to wirelessly transmit a detection signal to the receiver (4) to allow the receiver (4) to output a notification.

2. The stool detection device as claimed in Claim 1, wherein a length of said long board-member (57) is longer than a length of said short board-member (58) in the first direction.

3. The stool detection device as claimed in any one of Claims 1 and 2, **characterised in that** the transmitter module (6) is configured to use a wireless technology of Wi-Fi to transmit the detection signal to the receiver (4).

4. The stool detection device as claimed in any one of Claims 1 and 2, **characterised in that** the transmitter module (6) is configured to use a wireless technology of Bluetooth^{®} to transmit the soft stool detection signal to the receiver (4).

5. The stool detection device as claimed in any one of Claims 1 and 2, **characterised in that** said transmitter module (6) is configured to use a wireless technology of radio-frequency identification to transmit the soft stool detection signal to the receiver (4).

6. The stool detection device as claimed in any one of Claims 1 to 5, **characterised in that**:
said stool detector (5) further includes a connector (55), to which said the other end of said first electric wire (53) and said the other end of said second electric wire (54) are connected in a manner that said the other end of said first electric wire (53) and said the other end of said second electric wire (54) are insulated from each other; and
said transmitting module (6) includes an input port (61), and said connector (55) is inserted into said input port (61) so as to electrically connect said first electric wire (53) and said second electric wire (54) to said transmitting module (6).

7. The stool detection device as claimed in any one of Claims 1 to 6, **characterised in that**:
said positioning member (3) is a clip, and includes a first portion (31) that is partially inserted into said mounting seat (2), a second portion (32) that curvedly extends from said first portion (31), and a third portion that extends from said second portion (32);
when said positioning member (3) clips to the diaper (7), said first portion (31) is disposed on the inner surface (71) of the diaper (7), said second portion (32) extends across the through hole (73), and said third portion (33) abuts against the outer surface (72) of the diaper (7).

8. The stool detection device as claimed in any one of Claims 1 to 7, **characterised in that** said long board-member (57) is formed with said main body (56) of said stool detector (5) as one piece.

9. The stool detection device as claimed in any one of Claims 1 to 8, **characterised in that** said short board-member (58) is formed with said main body (56) of said stool detector (5) as one piece.

10. The stool detection device as claimed in any one of Claims 1 to 7, **characterised in that** said main body (56), said long board-member (57) and said short board-member (58) of said stool detector (5) are formed as one piece.

## Patentansprüche

1. Eine Stuhlerfassungsvorrichtung, die angepasst ist zum Erfassen und Melden einer Abscheidung von Stuhl (81) in einer Windel (7), die eine Innenoberfläche (71) und eine Außenoberfläche (72) aufweist, die mit einem Durchgangsloch (73) durch die Innenoberfläche (71) und die Außenoberfläche (72) gebildet ist, wobei die Stuhlerfassungsvorrichtung dazu angepasst ist, mit einem Empfänger (4) zu kommunizieren, und **gekennzeichnet ist durch**:
ein Positionierungsbauteil (3), das dazu angepasst ist, entfernbar an der Windel (7) angebracht zu sein und sich **durch** das Durchgangsloch (73) der Windel (7) hindurch zu erstrecken;
eine Befestigungsauflage (2), die an dem Positionierungsbauteil (3) in einer derartigen Weise befestigt ist, dass die Befestigungsauflage (2) im Inneren der Windel (7) angeordnet ist, wenn das Positionierungsbauteil (3) an der Windel (7) befestigt ist, und eine Befestigungsoberfläche (20), die dazu angepasst ist, von der Innenoberfläche (71) der Windel (7) weg zu zeigen, wenn das Positionierungsbauteil (7) an der Windel (7) befestigt ist, eine Angrenzoberfläche (23), die dazu angepasst ist, an die Innenoberfläche (71) der Windel (7) anzugrenzen, wenn das Positionierungsbauteil (3) an der Windel (7) befestigt ist, ein erstes Ende (21), das mit dem Positionierungsbauteil (3) verbunden ist, und ein zweites Ende (22) gegenüber von dem ersten Ende (21) in einer ersten Richtung parallel zu der Befestigungsoberfläche (20) aufweist;
ein Sendermodul (6), das entfernbar in einer derartigen Weise an dem Positionierungsbauteil (3) befestigt ist, dass das Sendemodul (6) außerhalb der Windel (7) angeordnet ist, wenn das Positionierungsbauteil (3) an der Windel (7) befestigt ist; und
einen Stuhldetektor (5), der an der Befestigungsoberfläche (20) der Befestigungsauflage (2) befestigt ist, und Folgendes aufweist:
einen Hauptkörper (56), der aus einem elektrisch isolierenden Material hergestellt ist, der benachbart zu dem ersten Ende (21) der Befestigungsauflage (2) ist, der eine erste Haftoberfläche (561) aufweist, die entfernbar an der Befestigungsoberfläche (20) der Befestigungsauflage (2) anhaftet, und der mit einem hydrophoben Material beschichtet ist, außer an der ersten Haftoberfläche (561),
ein langes Plattenbauteil (57), das aus einem elektrisch isolierenden Material hergestellt ist, das sich von dem Hauptkörper (56) in Richtung des zweiten Endes (22) der Befestigungsauflage (2) erstreckt, das eine zweite Haftoberfläche (571) aufweist, die entfernbar an der Befestigungsoberfläche (20) der Befestigungsauflage (2) anhaftet, und das mit einem hydrophoben Material beschichtet ist, außer an der zweiten Haftoberfläche (571),
ein kurzes Plattenbauteil (58), das aus einem elektrisch isolierenden Material hergestellt ist, das sich von dem Hauptkörper (56) in Richtung des zweiten Endes (22) der Befestigungsauflage (2) erstreckt, das von dem langen Plattenbauteil (57) beabstandet ist, das in einer zweiten Richtung, die senkrecht zu der ersten Richtung ist, dünner als der Hauptkörper (56) ist, das mit dem Hauptkörper (56) und dem langen Plattenbauteil (57) zusammenwirkt, um einen Aufnahmeraum (59) zu definieren, und das mit einem hydrophoben Material beschichtet ist,
eine erste leitfähige Lage (51), die in dem Aufnahmeraum (59) angeordnet ist,
eine zweite leitfähige Lage (52), die in dem Aufnahmeraum (59) angeordnet ist und die von der ersten leitfähigen Lage (51) beabstandet ist,
einen ersten elektrischen Draht (53), der dazu angepasst ist, sich **durch** das Durchgangsloch (73) der Windel (7) zu erstrecken, und bei dem ein Ende elektrisch mit der ersten leitfähigen Lage (51) verbunden ist und das andere Ende elektrisch mit dem Sendermodul (6) in einer entfernbaren Verbindung verbunden ist, und
einen zweiten elektrischen Draht (54), der dazu angepasst ist, sich **durch** das Durchgangsloch (73) der Windel (7) zu erstrecken, und bei dem ein Ende elektrisch mit der zweiten leitfähigen Lage (52) verbunden ist und das andere Ende elektrisch mit dem Sendermodul (6) in einer entfernbaren Verbindung verbunden ist,
wobei, wenn der Stuhl (81) in den Aufnahmeraum (59) des Stuhldetektors (5) gelangt und sowohl die erste leitfähige Lage (51) als auch die zweite leitfähige Lage (52) berührt, und dabei eine elektrische Verbindung zwischen der ersten leitfähigen Lage (51) und der zweiten leitfähigen Lage (52) einrichtet, das Sendermodul (6) dazu ausgebildet ist, aktiviert zu werden, um drahtlos ein Erfassungssignal an den Empfänger (4) zu übertragen, um zu ermöglichen, dass der Empfänger (4) eine Benachrichtigung ausgibt.

2. Die Stuhlerfassungsvorrichtung gemäß Anspruch 1, bei der eine Länge des langen Plattenbauteils (57) länger ist als eine Länge des kurzen Plattenbauteils (58) in der ersten Richtung.

3. Die Stuhlerfassungsvorrichtung gemäß einem der Ansprüche 1 und 2, die **dadurch gekennzeichnet ist, dass** das Sendermodul (6) dazu ausgebildet ist, eine WiFi-Drahtlostechnologie zu verwenden, um das Erfassungssignal an den Empfänger (4) zu senden.

4. Die Stuhlerfassungsvorrichtung gemäß einem der Ansprüche 1 und 2, die **dadurch gekennzeichnet ist, dass** das Sendermodul (6) dazu ausgebildet ist, eine Bluetooth^{®}-Drahtlostechnologie zu verwenden, um das weiche Stuhlerfassungssignal an den Empfänger (4) zu senden.

5. Die Stuhlerfassungsvorrichtung gemäß einem der Ansprüche 1 und 2, die **dadurch gekennzeichnet ist, dass** das Sendermodul (6) dazu ausgebildet ist, eine Hochfrequenzidentifikations-Drahtlostechnologie zu verwenden, um das weiche Stuhlerfassungssignal an den Empfänger (4) zu senden.

6. Die Stuhlerfassungsvorrichtung gemäß einem der Ansprüche 1 bis 5, die **dadurch gekennzeichnet ist, dass**:
der Stuhldetektor (5) ferner ein Verbindungselernent (55) aufweist, mit dem das andere Ende des ersten elektrischen Drahts (53) und das andere Ende des zweiten elektrischen Drahts (54) in einer derartigen Weise verbunden sind, dass das andere Ende des ersten elektrischen Drahts (53) und das andere Ende des zweiten elektrischen Drahts (54) voneinander isoliert sind; und
das Sendermodul (6) eine Eingangsöffnung (61) aufweist und das Verbindungselement (55) in die Eingangsöffnung (61) so eingeführt ist, um elektrisch den ersten elektrischen Draht (53) und den zweiten elektrischen Draht (54) mit dem Sendemodul (6) zu verbinden.

7. Die Stuhlerfassungsvorrichtung gemäß einem der Ansprüche 1 bis 6, die **dadurch gekennzeichnet ist, dass**:
das Positionierungsbauteil (3) eine Klemme ist und einen ersten Abschnitt (31), der teilweise in die Befestigungsauflage (2) eingeführt ist, einen zweiten Abschnitt (32), der sich von dem ersten Abschnitt (31) gekrümmt erstreckt, und einen dritten Abschnitt aufweist, der sich von dem zweiten Abschnitt (32) erstreckt;
wenn das Positionierungsbauteil (3) an der Windel (7) klemmt, der erste Abschnitt (31) an der Innenoberfläche (71) der Windel (7) angeordnet ist, der zweite Abschnitt (32) sich über das Durchgangsloch (73) hinweg erstreckt und der dritte Abschnitt (33) an die Außenoberfläche (72) der Windel (7) angrenzt.

8. Die Stuhlerfassungsvorrichtung gemäß einem der Ansprüche 1 bis 7, die **dadurch gekennzeichnet ist, dass** das lange Plattenbauteil (57) mit dem Hauptkörper (56) des Stuhldetektors (5) als ein Stück gebildet ist.

9. Die Stuhlerfassungsvorrichtung gemäß einem der Ansprüche 1 bis 8, die **dadurch gekennzeichnet ist, dass** das kurze Plattenbauteil (58) mit dem Hauptkörper (56) des Stuhldetektors (5) als ein Stück gebildet ist.

10. Die Stuhlerfassungsvorrichtung gemäß einem der Ansprüche 1 bis 7, die **dadurch gekennzeichnet ist, dass** der Hauptkörper (56), das lange Plattenbauteil (57) und das kurze Plattenbauteil (58) des Stuhldetektors (5) als ein Stück gebildet sind.

## Revendications

1. Dispositif de détection de selles adapté pour détecter et notifier la défécation de selles (81) dans une couche (7) qui présente une surface intérieure (71) et une surface extérieure (72), qui est formé avec un trou traversant (73) à travers la surface intérieure (71) et la surface extérieure (72), ledit dispositif de détection de selles étant adapté pour communiquer avec un récepteur (4) et étant **caractérisé par**:
un élément de positionnement (3) qui est adapté pour être monté de manière amovible sur la couche (7) et pour s'étendre à travers le trou traversant (73) de la couche (7);
un siège de montage (2) monté sur ledit élément de positionnement (3) de manière que ledit siège de montage (2) soit disposé à l'intérieur de la couche (7) lorsque ledit élément de positionnement (3) est monté sur la couche (7), et présentant une surface de montage (20) adaptée pour être disposée en s'éloignant de la surface intérieure (71) de la couche (7) lorsque ledit élément de positionnement (3) est monté sur la couche (7), une surface de venue en butée (23) adaptée pour venir en butée contre le surface intérieure (71) de la couche (7) lorsque ledit élément de positionnement (3) est monté sur la couche (7), une première extrémité (21) connectée audit élément de positionnement (3), et une deuxième extrémité (22) opposée à ladite première extrémité (21) dans une première direction parallèle à ladite surface de montage (20);
un module de transmission (6) monté de manière amovible sur ledit élément de positionnement (3) de manière que ledit module de transmission (6) soit disposé à l'extérieur de la couche (7) lorsque ledit élément de positionnement (3) est monté sur la couche (7); et
un détecteur de selles (5) monté sur ladite surface de montage (20) dudit siège de montage (2), et comportant
un corps principal (56) qui est réalisé en un matériau électriquement isolant, qui est adjacent à ladite première extrémité (21) dudit siège de montage (2), qui présente une première surface adhésive (561) adhérée de manière amovible à ladite surface de montage (20) dudit siège de montage (2), et qui est revêtu d'un matériau hydrophobe, sauf sur ladite première surface adhésive (561),
un élément de plaque long (57) qui est réalisé en un matériau électriquement isolant, qui s'étend dudit corps principal (56) vers ladite deuxième extrémité (22) dudit siège de montage (2), qui présente une deuxième surface adhésive (571) adhérée de manière amovible à ladite surface de montage (20) dudit siège de montage (2), et qui est revêtu d'un matériau hydrophobe, sauf sur ladite deuxième surface adhésive (571),
un élément de plaque court (58) qui est réalisé en un matériau électriquement isolant, qui s'étend dudit corps principal (56) vers ladite deuxième extrémité (22) dudit siège de montage (2), qui est distant dudit élément de plaque long (57), qui est espacé dudit élément de plaque long (57), qui est plus mince que ledit corps principal (56) dans une deuxième direction perpendiculaire à la première direction, qui coopère avec ledit corps principal (56) et avec ledit élément de plaque long (57) pour définir un espace de réception (59), et qui est revêtu d'un matériau hydrophobe,
une première feuille conductrice (51) qui est disposée dans ledit espace de réception (59),
une deuxième feuille conductrice (52) qui est disposée dans ledit espace de réception (59) et qui est distante de ladite première feuille conductrice (51),
un premier fil électrique (53) qui est adapté pour s'étendre à travers le trou traversant (73) de la couche (7), et qui présente une extrémité connectée électriquement à ladite première feuille conductrice (51) et l'autre extrémité connectée électriquement audit module de transmission (6) dans une connexion amovible, et
un deuxième fil électrique (54) qui est adapté pour s'étendre à travers le trou traversant (73) de la couche (7), et qui présente une extrémité connectée électriquement à ladite deuxième feuille conductrice (52) et l'autre extrémité connectée électriquement audit module de transmission (6) dans une connexion amovible,
dans lequel, lorsque les selles (81) arrivent dans ledit espace de réception (59) dudit détecteur de selles (5) et entrent en contact avec tant ladite première feuille conductrice (51) que ladite deuxième feuille conductrice (52) pour établir une connexion électrique entre ladite première feuille conductrice (51) et ladite deuxième feuille conductrice (52), ledit module de transmission (6) est configuré pour être activé pour transmettre sans fil un signal de détection au récepteur (4) pour permettre au récepteur (4) d'émettre une notification.

2. Dispositif de détection de selles selon la revendication 1, dans lequel une longueur dudit élément de plaque long (57) est plus longue qu'une longueur dudit élément de plaque court (58) dans la première direction.

3. Dispositif de détection de selles selon l'une quelconque des revendications 1 et 2, **caractérisé par le fait que** le module de transmission (6) est configuré pour utiliser une technologie sans fil de Wi-Fi pour transmettre le signal de détection au récepteur (4).

4. Dispositif de détection de selles selon l'une quelconque des revendications 1 et 2, **caractérisé par le fait que** le module de transmission (6) est configuré pour utiliser une technologie sans fil de Bluetooth^{®} pour transmettre le signal de détection de selles molles au récepteur (4).

5. Dispositif de détection de selles selon l'une quelconque des revendications 1 et 2, **caractérisé par le fait que** ledit module de transmission (6) est configuré pour utiliser une technologie sans fil d'identification par radiofréquence pour transmettre le signal de détection de selles molles au récepteur (4).

6. Dispositif de détection de selles selon l'une quelconque des revendications 1 à 5, **caractérisé par le fait que**:
ledit détecteur de selles (5) comporte par ailleurs un connecteur (55), auquel sont connectées ladite autre extrémité dudit premier fil électrique (53) et ladite autre extrémité dudit deuxième fil électrique (54) de manière que ladite autre extrémité dudit premier fil électrique (53) et ladite autre extrémité dudit deuxième fil électrique (54) soient isolées l'une de l'autre; et
ledit module de transmission (6) comporte un orifice d'entrée (61), et ledit connecteur (55) est inséré dans ledit orifice d'entrée (61) de manière à connecter électriquement ledit premier fil électrique (53) et ledit deuxième fil électrique (54) audit module de transmission (6).

7. Dispositif de détection de selles selon l'une quelconque des revendications 1 à 6, **caractérisé par le fait que**:
ledit élément de positionnement (3) est un clip et comporte une première partie (31) qui est partiellement insérée dans ledit siège de montage (2), une deuxième partie (32) qui s'étend de manière courbée à partir de ladite première partie (31), et une troisième une partie qui s'étend depuis ladite deuxième partie (32);
lorsque ledit élément de positionnement (3) est accroché à la couche (7), ladite première partie (31) est disposée sur la surface intérieure (71) de la couche (7), ladite deuxième partie (32) s'étend à travers le trou traversant (73), et ladite troisième partie (33) vient en butée contre la surface extérieure (72) de la couche (7).

8. Dispositif de détection de selles selon l'une quelconque des revendications 1 à 7, **caractérisé par le fait que** ledit élément de plaque long (57) est formé avec ledit corps principal (56) dudit détecteur de selles (5) comme une seule pièce.

9. Dispositif de détection de selles selon l'une quelconque des revendications 1 à 8, **caractérisé par le fait que** ledit élément de plaque court (58) est formé avec ledit corps principal (56) dudit détecteur de selles (5) comme une seule pièce.

10. Dispositif de détection de selles selon l'une quelconque des revendications 1 à 7, **caractérisé par le fait que** ledit corps principal (56), ledit élément de plaque long (57) et ledit élément de plaque court (58) dudit détecteur de selles (5) sont formés comme une seule pièce.
